# EUROPEAN PATENT SPECIFICATION

(11) **EP 2 689 036 B1**
(45) Date of publication and mention of the grant of the patent: **20.12.2017**
(21) Application number: 12765854.0
(22) Date of filing: 26.03.2012
(51) Int. Cl.: G01N 33/48

(54) **METHODS OF DIAGNOSING AND TREATING INTESTINAL GRANULOMAS AND LOW BONE DENSITY IN INFLAMMATORY BOWEL DISEASE**
VERFAHREN ZUR DIAGNOSE UND BEHANDLUNG INTESTINALER GRANULOME UND NIEDRIGER KNOCHENDICHTE BEI ENTZÜNDLICHEN DARMERKRANKUNGEN
MÉTHODES DE DIAGNOSTIC ET DE TRAITEMENT DES GRANULOMES INTESTINAUX ET DE LA FAIBLE DENSITÉ OSSEUSE DANS LA MALADIE INTESTINALE INFLAMMATOIRE

(30) Priority: 25.03.2011 US 201161467779 P; 25.03.2011 US 201161467881 P
(43) Date of publication of application: 29.01.2014
(73) Proprietor: Cedars-Sinai Medical Center, Los Angeles, CA 90048 (US)
(72) Inventor: McGOVERN, Dermot P., Los Angeles California 90049 (US); DUBINSKY, Marla C., Los Angeles California 90048 (US); TAYLOR, Kent D., Ventura California 93004 (US); TARGAN, Stephan R., Santa Monica California 90402 (US); ROTTER, Jerome I., Los Angeles California 90064 (US)
(74) Representative: HGF Limited
(86) International application number: PCT/US2012/030614
(87) International publication number: WO 2012/135144

(56) References cited:
- US-A1- 2004 181 048
- US-A1- 2010 015 156
- US-A1- 2010 240 077
- ONNIE CLIVE M ET AL: "Diverse effects of the CARD15 and IBD5 loci on clinical phenotype in 630 patients with Crohn's disease", EUROPEAN JOURNAL OF GASTROENTEROLOGY AND HEPATOLOGY, LIPPINCOTT WILLIAMS AND WILKINS, UK, vol. 20, no. 1, 1 January 2008 (2008-01-01), pages 37-45, XP009180627, ISSN: 0954-691X
- BRINAR M ET AL: "P217 - Genetic variants in autophagy related genes and granuloma formation in patients with Crohn's disease", JOURNAL OF CROHN'S AND COLITIS, ELSEVIER BV, NL, vol. 3, no. 1, 1 February 2009 (2009-02-01), page S96, XP025961627, ISSN: 1873-9946 [retrieved on 2009-02-01]
- QUINTON J F ET AL: "Anti-Saccharomyces cerevisiae mannan antibodies combined with antineutrophilcytoplasmic autoantibodies in inflammatory bowel disease: prevalence and diagnostic role", GUT, BRITISH MEDICAL ASSOCIATION, LONDON, UK, vol. 42, no. 6, 1 June 1998 (1998-06-01), pages 788-791, XP009087984, ISSN: 0017-5749
- D G FORCIONE: "Anti-Saccharomyces cerevisiae antibody (ASCA) positivity is associated with increased risk for early surgery in Crohn's disease", GUT, vol. 53, no. 8, 1 August 2004 (2004-08-01) , pages 1117-1122, XP055145792, ISSN: 0017-5749, DOI: 10.1136/gut.2003.030734
- NOBUHIDE OSHITANI1 ET AL: "Cross-reactivity of yeast antigens in human colon and peripheral leukocytes", THE JOURNAL OF PATHOLOGY, vol. 199, no. 3, 1 March 2003 (2003-03-01) , pages 361-367, XP055145795, ISSN: 0022-3417, DOI: 10.1002/path.1276
- DATABASE BIOSIS [Online] BIOSCIENCES INFORMATION SERVICE, PHILADELPHIA, PA, US; May 2011 (2011-05), MODIANO NIR ET AL: "Intestinal Granulomas in Crohn's Disease: Association With Patient Characteristics, Serologic Markers, and Genetics", XP002730930, Database accession no. PREV201100403923 & GASTROENTEROLOGY, vol. 140, no. 5, Suppl. 1, May 2011 (2011-05), page S484, DIGESTIVE DISEASE WEEK 2011; CHICAGO, IL, USA; MAY 07 -10, 2011 ISSN: 0016-5085
- FRANKE ET AL.: 'Genome-wide meta-analysis increases to 71 the number of confirmed Crohn's disease susceptibility loci' NAT. GENET. vol. 42, no. 12, December 2010, pages 1118 - 1125, XP055137311
- DATABASE GENBANK 30 August 2012 BIRREN ET AL., XP055137313 Retrieved from C021483, accession no. N N N Y
- DATABASE GENBANK 30 August 2012 JOHNSON, XP055137315 Retrieved from L357149, accession no. N N N Y
- DATABASE GENBANK 30 August 2012 JOHNSON, XP055137319 Retrieved from L357075, accession no. N N N Y
- DATABASE GENBANK 30 August 2012 KAUL ET AL., XP055137322 Retrieved from C107626, accession no. N N N Y
- DATABASE GENBANK 30 August 2012 SULSTON ET AL., XP055137324 Retrieved from C093601, accession no. N N N Y
- DATABASE GENBANK 30 August 2012 BIRREN ET AL., XP055137325 Retrieved from C026826, accession no. N N N Y
- DATABASE GENBANK 30 August 2012 BIRREN ET AL., XP055137327 Retrieved from C105243, accession no. N N N Y
- BARRETT ET AL.: 'Genome-wide association defines more than 30 distinct susceptibility loci for Crohn's disease' NAT. GENET. vol. 40, no. 8, August 2008, pages 955 - 962, XP055033904
- ISRAELI ET AL.: 'Anti-Saccharomyces cerevisiae and antineutrophil cytoplasmic antibodies as predictors of inflammatory bowel disease' GUT vol. 54, no. 9, September 2005, pages 1232 - 1236, XP055137330

### SEQUENCE LISTING

<110> CEDARS-SINAI MEDICAL CENTER
   ROTTER, Jerome I.
   TARGAN, Stephan R.
<120> METHODS OF DIAGNOSING AND TREATING INTESTINAL GRANULOMAS AND LOW BONE DENSITY IN INFLAMMATORY BOWEL DISEASE
<130> 67789-312WO0
<150> US 61/467,779
   <151> 2011-03-25
<160> 15
<170> PatentIn version 3.5
<210> 1
   <211> 701
   <212> DNA
   <213> Homo sapiens
<400> 1
   tctcctgtct ccaatgtggt ttttgttttg ttttgttttg ttttttgaga cagtcttgct      60
   ctgtcgccca ggctggagtg cagtggcgta atctcagctc actgcaacct ctgcctcctg     120
   ggttcaagcc attctcctgc ctcagcctcc tgagtagctg ggattacagg tgcacaacac     180
   cacgcccagc taatttttgt atttttagta gagacggggt ttcaccatgt tggccaggat     240
   ggtctcaatc tcttgacctc gtgatctgcc cgcctctgcc tcccaaagtg ctgggattac     300
   aggcatgagc caccgcgccc ggccactgac gtgttgttaa aagagccgtg actgaagtcc     360
   caccacccag ctttgcaagt gctataatct gacctcagtt ccaaatacct atttaaacgt     420
   caggaacaaa gggctttttt ttattttttc aaaacatcag tctttaagct ttaaaaatgt     480
   ttccatggag agtaactcag ygtcaagaga tgtgcagaaa atccaaagaa gggtaaggtt     540
   gcccctggta tcaaggaaat aagaaccaga agtcatcagg acagtgaaga tatttagcaa     600
   agaaatgtaa gtgggaaaac atgtaataaa gttagttttg tgatttaata aataaataaa     660
   atgtttgcgt atgcatcgct tcccagaagc agccaggcat c                          701
<210> 2
   <211> 623
   <212> DNA
   <213> Homo sapiens
<400> 2
   ctggtctgca gttggaagga aggcaatgct atcacagaca gttcactcct aaaccacaag 60
   ctactgcagc cttctcaagc atgagccaag ccccattaat ctgattcaaa acctacagag 120
   attgcatatc aatttctcaa tgtaatccca ttggagtttt tcatgctaga cttgggatta 180
   ggggcaattg ccccctttac acacactctc tcgtttcata ggttgaactc aacaagatag 240
   ctcactgcaa agaagtcctc tccaaattct acttatcacg cccaataata gccacttttt 300
   agatcgttaa aaagatctaa aaaaatcgtg tggcttgaga gtcattcaat cagtgaactg 360
   acttacaatt cattttagaa ggtgtcatct agtgtctttg gcctcaattg aaagacagga 420
   caagtcccat tttaatgtcc tgttatagga ataacctaat gaaatatttg tacatacaca 480
   ggttgaggtt agatggcaac aagagtgttg taacatagga gctgcaatat ygacagcttg 540
   cttaatccaa atagaaagtg attctgctaa tacaggcacc ttcaatttag ctcaccctta 600
   gaaatgaatt gacattattc ttt 623
<210> 3
   <211> 501
   <212> DNA
   <213> Homo sapiens
<400> 3
   ttctgtattc ttagctaaaa tcccatctca aatcacatct tttgaaagaa gacttctgtg 60
   agtatgatta ggggcttgaa ggtgccatct ataactcctc cctgtgaaga ttctggccaa 120
   ttaagtttag gtaccaaaaa taaaaaaaaa aaacccacaa aaaaacaata aataggaatg 180
   gaagcatcac tttatttgtt tgggaaagca catgtaacca tccagagctc tgataatggc 240
   tggaatagca mgacgctgga gtggagaatt ctatttccta gcccagatct agatcttgaa 300
   acttacattc ttaggtgtgc catgtgtgag aaagagcaaa aagaagagag gaatgaagag 360
   agatggcctt caaagctgac tgagcctgct gcttagaagg tgacctcaag ccttcccaca 420
   ttagaaataa tgaagataat ttctttcatc ctacactaat cattctcttc ttcaaacacc 480
   attttaactt agaggtaaca t 501
<210> 4
   <211> 601
   <212> DNA
   <213> Homo sapiens
<400> 4
   aaagtacagg gagaatttgg agttccttcc aaactccggg tggaagaaaa ggtccttgta 60
   caactctggg gtttcagcag cagaaattaa tgtcttctgt agtccaactc atgaactcaa 120
   ctcagttctg gtaaaaatca gtccccaggc ctcacaataa ggtataggag agagactctg 180
   actgatccag cctgggcaag atggatacct tgcctggatt gtccactgat ggaccaagac 240
   acagagtcac atagtatggt cttgaccagc agagggttga acagagtagc aaggctagaa 300
   mtggaaatgc tgacatcctt ttgagccagg cagtcagtga agagagggct gcagaaagaa 360
   gttttacctg tagcccagtg ttgctaataa aatgttgaaa aactatctga gggcagccag 420
   acctgttccc acaagtgata gctggttttc atttggggat cttcactttg tgacaagccg 480
   gatttctatt tccatcagat aaaggctggc aataatgaac atcttcctga taactctcat 540
   cacggttttc caaggagctg aaaatggtaa ttaatgcaaa agctgtgttt attggttcgt 600
   g 601
<210> 5
   <211> 1001
   <212> DNA
   <213> Homo sapiens
<400> 5
   ggagtctccc tctgttgccc aagctggatt gcaatggcat gatctcggct cactgcaacc 60
   tccacctcct gggttccacc catcctcctg cctcagcccc caagtagctg ggactacagg 120
   tgtgcgccac cgcacctggc taatttttgt atttttagta gcgattgggt ttctccatgt 180
   tggccaagct ggtttcaaac tcctgacttc agcctcccaa agtgctggga ttacatatgt 240
   gagtcaccct gcccagcctg tcttggtttt ttgatcgtta aataatctcc ctctcctctt 300
   gctttctctg ttttctcact ctgatataat tactgatgga tcttctaatt tgcttatctt 360
   gtttgctgct actcctgtct cagtgtcctc ttctgctttg gggaagattt ttttcaacgt 420
   cacccttcat atttagtttt acggttgtgc catctttgtt tcaatatcca ggaggctctt 480
   tcttgttctc tgaatattaa yttttttatc accctcattc ttacatgcat cttgactctc 540
   accttttctg aggatattaa ctaatagcaa gtagtttgtt ttcgacattt tcttctgttt 600
   ccttcaagtt actcttattt gggctctgca tttcatctta gaggcttggc ctctgatgct 660
   tagtgatccc tggctattca tatttagaca taaaacacta aatgtttctt gtaagttctc 720
   agtgctggtg ggcttcaaaa tagggtgacc gtgagcgtta attttatgtg tcaacatgac 780
   tggactacag gatacacaga catttgatca aacattgttc ttagtgtttt tggatgagtt 840
   taatgtttaa atccataaac tgagtaaagc aaattgcccc ctctggaatg tgatgggcct 900
   tatccaatct caggcctgaa tagaaaaaaa gaccgaccct cctgcaagta agagagaacg 960
   cctcctgcct gagaaggggt tcagaacagg gacattgttt t 1001
<210> 6
   <211> 1948
   <212> DNA
   <213> Homo sapiens
<400> 6
   ttctcaaact ctatgggggc agattttatg tactgaagtg tttcatgatc tactgtgtgt 60
   tggactgtta atttgttctt tttctttttc taaaattaat ttcccttctc ccttccttcc 120
   ttccttcttt ccttccttcc ttccctcctt cccttccttc cttccttccc ttccttcctt 180
   tcctccttca ttcctttcct ttcctttctt ccttccttct tcccttcttt cctttccttt 240
   ttctcttctc ttctcttctc ttctctcttt gagatggagt ctcgcattgt tgcttaggct 300
   ggtctaagct ctggcctcaa gcagtcctcc tgccttggcc tcccaaagtg ctgggattac      360
   agacaggagc cactgtacct ggcttaactt gttttttcaa tgagactttg ggacaacata      420
   ctgagtttgg gggttgtgtg agtcagacag attagcacta tgctgtaatc ataaataaca      480
   cctaaatctc acctgtttaa atcagtaaag agttattttt tcttttgctg caatttcatc      540
   cccgctttgg caggggaatc tttttcacgt ctcatgcaga tatggaggct gacagggacc      600
   tcactctctg ttactggtct ccatggcagg aagaaggaag ggaatgtgat aaattgtatg      660
   tggaattttt gtttgtttca ttttgctttg ctattattca actttcttga ggtataattg      720
   aagtatcata aactgcatat atttaaaatg taccttttga tgcattttga gatatgcatg      780
   tacctttgat atcctcacca tgatgaagag aataaacatt ttcataaccc ccaaaaggtt      840
   ccttgtttca catgcctctc tccactcctg tcttcatgca accagggacc tgctatctgt      900
   caatgtaggt tgatctgcat gttccggaat tttttttttt tttttaataa acggaagcac      960
   acagcactta ctcttcttct actggcttct ttcatgcagc tcaatgattt gaggaatcac     1020
   tcatgttgta tgtgcctcaa cagttcattc tgttttattg ctgagtagta ttccattgta     1080
   tggctatacc acaatttgtt tatctattcc tgcacatgga aagttggttt gtttctggtt     1140
   trtaatgatc acaaatcaag ctgataagaa catttgggta caagtctttg tgtagacttg     1200
   tgcttttatt tatcttggat gcatacttag gagtgaagat gtcccaaagt ggttgtacca     1260
   tttcacattc ccttagcagt ttataagagt tccagttgct ccacatcttc accaacacat     1320
   ggtatgatca gttgaaaaac ttttcccagc actttgggag gccgaggtgg gtggatcaca     1380
   aggtcaggag atcgagacca tcctggctaa cacagtgaaa ccctgtctct attaaaaata     1440
   caaaaaatta gctgggcatg gtggcaggtg cctatagtcc cagctacttg ggaggctgag     1500
   gcaggagaat ggcgtgaacc cgggaggcgg agcttgcagt gagctgagat tgcgccactg     1560
   cactccagcc tggaagacag agcgaggctc catctcgaaa caacaaaaaa cacaacaaaa     1620
   aactgttcac ccttcaaata ggtggatagt ggtatctcag tgtagtttta atttgcattt     1680
   tcctaatgat taattatatt gagcattttt aatgtgttta ttggctgtat tgtggagtat     1740
   ccttgggtgc tgtgctccgg ggtttgctct ggtgtgtcaa cttggtagaa tgacttttat     1800
   ccaatttttt tccagtgagg cttctagtta gtacaatatc tacacactgt agcactagtt     1860
   agcatccccg ttcctacttt gtttttatct gaaccagttt agcccttcca tttctctcct     1920
   gacctgtggc tagtttatgg gctcccta                                        1948
<210> 7
   <211> 1125
   <212> DNA
   <213> Homo sapiens
<400> 7
   cctaagagag aaaggtgtca ttgaaagaat tccgtctgta aaattatttt ggggtttctt      60
   aaattcacat agtcttgtac atagtttaat gtacatggcc ttggcattgt gtaaaatgtt     120
   atttggacac tctggaagaa gaggatagca aatttgattc tagggttata agactggagg     180
   gttaattata atataaatca rtcaggtaat tgggagaaga ttggaggaaa tttgatacat     240
   gtttatgtgt ttatcaagtg attgcatctg cttttcactc tctgctgcca ccatctggcc     300
   ccaaaacctt tgtatgagcc acaatagtca catccctggg acatctttgt gccccctcca     360
   gtctcttttc cacacggcag ccaatatcat ttcttatgat tctctactgg ttcatgtcat     420
   tcctacactt ggcatcactc attagttgga ctttggataa acagaaagca tcaagctctt     480
   gcctcctttc cagcttcatc tcatgtcttc cctcactcgt aacgctctgc ttatgccagc     540
   cttctctctg gcccttgact atatggagtt ctttccagcc tcagggtatt tgcaacctgc     600
   tgttctgcta cacaggacat ctgccattca cactttctat ctctcactcc tgctcatccc     660
   ttagatctca gctaaacata actttcttgg atggcactcc tttaaagccc agccacccga     720
   ccccagaatt taaattggcc tctccccatc cttaatctaa tttccaacag ggcatactga     780
   ccttttcctt tgtataactt attataattt gtggtgtcat gttttctata tgcttgttta     840
   acctctgtct tccccaatag acagttaact ttataaagcc atggacggtg tccatctttt     900
   tttttttttt aagtgctgta ctctgcctca gacaagatgt ggttggtgct taattaatag     960
   ttgttgaatc aatgattaca tcaacaaacg aacacttagt atgtgacaag cactgggcta    1020
   agtgcataat actcatgatt ttatttaatc ctcacaataa ccttttgagt taattatcat    1080
   taatgtcatt cacaaatgag gaaataatac ttcttcatgc agtga                   1125
<210> 8
   <211> 605
   <212> DNA
   <213> Homo sapiens
<400> 8
   ttgattatgt ttttccaggt tcactttctt ccttgggatg tcacagaact gaactctatc        60
   agaaatatgc tagggcatgt tttattctct tctgcatcct ctgtcagaaa cagccaacaa       120
   gtgccatttt caagtcgcta cagatctcac ctagaccctt ggaaatatca ggttctgctg       180
   gattctgatc aagagtgccc acgcagtaac ttagaatctg accagcatgc caggactcac       240
   atctagctct tcccaaaggc acacgtgggc agggattctc aaatcatctg gggatgtcga       300
   taaaccacag atgytgactc agtgagtgtg gggtgaggac tgacactctt aatttccaaa       360
   agctcccagg tgccactgtg ggtctttggg actgcatttt gaatggaaag gtgctagggt       420
   atttttaatg acatcctaac gaaattatgc cattagggat ttaactgtgt attccacagc       480
   tttactttcc ttttcaattt aacctcttta gttcaacaat gtaacacttg tggaactcca       540
   caaattagga tgcaaaatga acaagaaaag ggatttagtt ttaactgaga tgtcccttca       600
   caaga                                                                   605
<210> 9
   <211> 701
   <212> DNA
   <213> Homo sapiens
<400> 9
   tctcctgtct ccaatgtggt ttttgttttg ttttgttttg ttttttgaga cagtcttgct        60
   ctgtcgccca ggctggagtg cagtggcgta atctcagctc actgcaacct ctgcctcctg       120
   ggttcaagcc attctcctgc ctcagcctcc tgagtagctg ggattacagg tgcacaacac       180
   cacgcccagc taatttttgt atttttagta gagacggggt ttcaccatgt tggccaggat       240
   ggtctcaatc tcttgacctc gtgatctgcc cgcctctgcc tcccaaagtg ctgggattac       300
   aggcatgagc caccgcgccc ggccactgac gtgttgttaa aagagccgtg actgaagtcc       360
   caccacccag ctttgcaagt gctataatct gacctcagtt ccaaatacct atttaaacgt       420
   caggaacaaa gggctttttt ttattttttc aaaacatcag tctttaagct ttaaaaatgt       480
   ttccatggag agtaactcag ygtcaagaga tgtgcagaaa atccaaagaa gggtaaggtt       540
   gcccctggta tcaaggaaat aagaaccaga agtcatcagg acagtgaaga tatttagcaa       600
   agaaatgtaa gtgggaaaac atgtaataaa gttagttttg tgatttaata aataaataaa       660
   atgtttgcgt atgcatcgct tcccagaagc agccaggcat c                           701
<210> 10
   <211> 1948
   <212> DNA
   <213> Homo sapiens
<400> 10
   ttctcaaact ctatgggggc agattttatg tactgaagtg tttcatgatc tactgtgtgt 60
   tggactgtta atttgttctt tttctttttc taaaattaat ttcccttctc ccttccttcc 120
   ttccttcttt ccttccttcc ttccctcctt cccttccttc cttccttccc ttccttcctt 180
   tcctccttca ttcctttcct ttcctttctt ccttccttct tcccttcttt cctttccttt 240
   ttctcttctc ttctcttctc ttctctcttt gagatggagt ctcgcattgt tgcttaggct 300
   ggtctaagct ctggcctcaa gcagtcctcc tgccttggcc tcccaaagtg ctgggattac 360
   agacaggagc cactgtacct ggcttaactt gttttttcaa tgagactttg ggacaacata 420
   ctgagtttgg gggttgtgtg agtcagacag attagcacta tgctgtaatc ataaataaca 480
   cctaaatctc acctgtttaa atcagtaaag agttattttt tcttttgctg caatttcatc 540
   cccgctttgg caggggaatc tttttcacgt ctcatgcaga tatggaggct gacagggacc 600
   tcactctctg ttactggtct ccatggcagg aagaaggaag ggaatgtgat aaattgtatg 660
   tggaattttt gtttgtttca ttttgctttg ctattattca actttcttga ggtataattg 720
   aagtatcata aactgcatat atttaaaatg taccttttga tgcattttga gatatgcatg 780
   tacctttgat atcctcacca tgatgaagag aataaacatt ttcataaccc ccaaaaggtt 840
   ccttgtttca catgcctctc tccactcctg tcttcatgca accagggacc tgctatctgt 900
   caatgtaggt tgatctgcat gttccggaat tttttttttt tttttaataa acggaagcac 960
   acagcactta ctcttcttct actggcttct ttcatgcagc tcaatgattt gaggaatcac 1020
   tcatgttgta tgtgcctcaa cagttcattc tgttttattg ctgagtagta ttccattgta 1080
   tggctatacc acaatttgtt tatctattcc tgcacatgga aagttggttt gtttctggtt 1140
   trtaatgatc acaaatcaag ctgataagaa catttgggta caagtctttg tgtagacttg 1200
   tgcttttatt tatcttggat gcatacttag gagtgaagat gtcccaaagt ggttgtacca 1260
   tttcacattc ccttagcagt ttataagagt tccagttgct ccacatcttc accaacacat 1320
   ggtatgatca gttgaaaaac ttttcccagc actttgggag gccgaggtgg gtggatcaca 1380
   aggtcaggag atcgagacca tcctggctaa cacagtgaaa ccctgtctct attaaaaata 1440
   caaaaaatta gctgggcatg gtggcaggtg cctatagtcc cagctacttg ggaggctgag 1500
   gcaggagaat ggcgtgaacc cgggaggcgg agcttgcagt gagctgagat tgcgccactg 1560
   cactccagcc tggaagacag agcgaggctc catctcgaaa caacaaaaaa cacaacaaaa 1620
   aactgttcac ccttcaaata ggtggatagt ggtatctcag tgtagtttta atttgcattt 1680
   tcctaatgat taattatatt gagcattttt aatgtgttta ttggctgtat tgtggagtat 1740
   ccttgggtgc tgtgctccgg ggtttgctct ggtgtgtcaa cttggtagaa tgacttttat 1800
   ccaatttttt tccagtgagg cttctagtta gtacaatatc tacacactgt agcactagtt 1860
   agcatccccg ttcctacttt gtttttatct gaaccagttt agcccttcca tttctctcct 1920
   gacctgtggc tagtttatgg gctcccta 1948
<210> 11
   <211> 622
   <212> DNA
   <213> Homo sapiens
<400> 11
   aagattgtaa ggcagagaaa agaatgctga gccatgagaa tggtaagagg ctgatcaatg    60
   gtgttgtggg acatgctaag aggtcttcac ttcgctatat tggggatagg aagcagcaat   120
   gtgctaagac ctacctgtca gcaaatatta ctgccatggt cccaaggcct taggcatgat   180
   agagctcaat aaatgctggg tgaatgaaca aataaatgaa tgcataagcc atgaaataat   240
   tggataaaat tctctaggtg cataaggaga atgaattaaa gcagtcaagg ctagaagtaa   300
   gaagaccatt taggaggtta ttgcatataa gaaatgctaa ggtatgaact ayagcagtgt   360
   ctgtaggact agaaaggagg gaacaaattc aggagcaatt cagaaaacag aattgataga   420
   gtcacagtcc tcacatggcc accagcttat cttcctgaaa gaacattgtt tattaggcca   480
   tttggctgaa caaaaacgtt cagcagtttc ccacaccttg tagaatatac atactgtaaa   540
   gtttgctacc tacattcacg gtccttcatc agcttctagc tctacatctg ccccagaata   600
   actattcatc aaatgctgaa ta                                            622
<210> 12
   <211> 914
   <212> DNA
   <213> Homo sapiens
<400> 12
   ttgaaacaaa gaaagtagag agaatattat agtgaactcc cacaaatcta gaaacaagct      60
   ttaatcattg tgaacatgct ttcaatcttt tctaatcacc ccacttgttt ttattgtatt     120
   tacttattta tatattttaa ttaatttttg ctgcaggatt ttaaagcaaa ttccagatat      180
   cataccacct ctccactgaa accagtatgt tgtcctaaat gaaaatcata ttaaaatttt     240
   acataacaat atggaattaa cctaacaatt ttgaccttaa tcacttagta ttatttgaat     300
   agtatttaat atctgcttca tatctgaaag tttccaattg aatcaaacaa aatattcttc     360
   ttataccata tttgtttaat tcagcattta aataagcaca agtattaatt ttggttgtta     420
   tgtcttctac atctatttta ctctccattt tttttcttct ttaatcatca aaaccagccc     480
   taaagaatgt ccaactttcc rcatttagtt tattgcttct tccttatatg agttaacttg     540
   ttcctctatt tcttccagat gttgcaaaag tagttagacc tagaaaatta attagctaac     600
   agttcaatta cgtctgtaca tgataaattt tagaggccat gcctcacact ctccatttca     660
   tcacattttg aggcacttat gcatctgtgg tctcttttgg tgatgctgat atcactgggt     720
   aacgtgcagt ctgaactttt ctcataacat tccccatcaa ctcttcacct aatgcttaca     780
   tcatccactg atccatcgaa tgttgtctaa attcattatt acattacaga tcgcaataca     840
   gtatttttct aattttatca tcccttatgg agtcttttcc aaaaattaga aagttaacct     900
   aaaagaaaac tcag                                                       914
<210> 13
   <211> 582
   <212> DNA
   <213> Homo sapiens
<400> 13
   ggcagaggag gtccttcgaa gctctctggg agcacaagcc tctgagcagg gatctgggtg      60
   aggggagcaa ggggctctgt gaagacccag ggaccagatt ccaggcagaa gccctcagag     120
   cagaagtggg attgaagggc tccaagagca gaataaagac cagtgtagcc agactgggga     180
   gaggggaggc cccatctcac atgggttgct tctgaatgtg atgtggggca gtggcctgct     240
   atgatgktct tttaaaaagc actagaactg ttgaatgaaa tcctgatgcc aggagggcca     300
   gggtggaatt ggggagccca ctgaggctgt ggcaggagtc tgggaagagt ctgagagggg     360
   tgatgaggca gggaggtggc agatttggga gaaacgtagg aggcagagat acaaggacca     420
   gcagaaggga acagaggagc aaggagagac ctgcttacag ctgagccatc ggggagagct     480
   ggtgccttct gctgaagtag gatgaacatg tcttgggggg aaatcaagac ttctgctttt     540
   taaagttaag tgatcagggg tgggtggcag agtctcgacc ta                        582
<210> 14
   <211> 1001
   <212> DNA
   <213> Homo sapiens
<400> 14
   ctcctatgcc ctgatgggga actctctaga cagacaaatg aatagctgag gcttctcatc      60
   acccatatac ccacattctc tctctctttt tttttttttt tttaaacaga gtctccttct     120
   gttgcccagg ctggagtaca gcctgggctc actggatcct ctgcctcctg ggttcaagtg     180
   attctcctgc ctcagtctct caagtaggtg ggactacagg tgcatgccac caggcccgac     240
   taatttttgt atttttagta gagacggggt ttcaccatga tggccaggtt ggcctcgaac     300
   tcctgacctc aagtgatcct cccaccttag cctcccaaag tgctaggatt acaggcgtga     360
   gccaccatgc ccggctgcac actctcttac ctttcccaca ccttgaaatt tccctcactg     420
   cccaccctaa atacagtacc atgagacgca aatcttagat tagatttcaa aggaagggca     480
   gtgtcactta gttccactaa rgcctacctg tatcctctgt ggcctagact ggaggtggat     540
   atggggagac tgatttccaa gatagacata gctctgttat cttcactccc tgagtccaag     600
   cccagctgaa tacctaagtg gcttctcatt cttttttttt tttttttgga gacggagtct     660
   tgccctgtcg cccaggctgg agtgcagtgt catgatcttg gctcactgca acctctgcct     720
   cccaggttca agcaattctc ctgcctcagc ctcttgagta gctgggatta caggtatgtg     780
   ccaccccacc tggctaattt ttgtattttt agtagagaca gggtttcacc atgttggccg     840
   ggatggtctc gatctcctga cctcgtgatc cacctgtctc ggcctcccaa agtgctggga     900
   ttacagatgt gagccaccac gcccagccgt ggcttctcat tcttatcctt agagattgct     960
   ggcatccccg gcctcttacc actcttccca tccttccatc a                        1001
<210> 15
   <211> 753
   <212> DNA
   <213> Homo sapiens
<400> 15
   atacatgaga tttctcatat atatatattt atatgacatc atgagattat agatctaata      60
   tataatttta ttatataatg ttatatatta tatttattat atataataat atatatttat      120
   aatatataat ctcatgatgt catgtatatc tgataatcac tatgtgatat atgtgctcac      180
   acatacaaac acattttttt atgttgttat atgtatttca tattataata tgtatgtatt      240
   cacaaaattt acccaaaagt gtattttccc tagttccata tctttataac ttgggtgcat      300
   tctgatgtac tctagtctat acttttttgt atttcatttc aaaaaatgct tgttataact      360
   gaatgcattg attttatatg ctataatggg tctacccata gactgaaaaa tttggctcta      420
   gttgaagttt ctcaaaaatc agtgtgtatt agaaatactt gggaagcttt ttacaaagct      480
   aggttcctga ggcccctccc yctaagattc tgacttgttg catcatagaa aaggcccagg      540
   aacctacatt ttttggctca atatatggaa aataaaaaca acagtgctca gtagacggtg      600
   ctaaggtcca tatgagaagt tcacacagtg tgttaaatcc atgttcagag tgaagagtgt      660
   ttgctgtcct gaacagagtc tgcactcctc caactgcacc tctcaatggg tgattatttc      720
   aaagatgtca agaaagaaaa gccaactcat aaa                                   753

## Description

The invention relates to the field of genetics and medicine. More specifically, the invention relates to methods of diagnosing and treating inflammatory bowel disease including ulcerative colitis and Crohn's disease.

### BACKGROUND

The following description includes information that may be useful in understanding the present invention. It is not an admission that any of the information provided herein is prior art or relevant to the presently claimed invention, or that any publication specifically or implicitly referenced is prior art.

Crohn's disease (CD) and ulcerative colitis (UC), the two common forms of idiopathic inflammatory bowel disease (IBD), are chronic, relapsing inflammatory disorders of the gastrointestinal tract. Each has a peak age of onset in the second to fourth decades of life and prevalences in European ancestry populations that average approximately 100-150 per 100,000 (D.K. Podolsky, N Engl J Med 347, 417 (2002); E.V. Loftus, Jr., Gastroenterology 126, 1504 (2004)). Although the precise etiology of IBD remains to be elucidated, a widely accepted hypothesis is that ubiquitous, commensal intestinal bacteria trigger an inappropriate, overactive, and ongoing mucosal immune response that mediates intestinal tissue damage in genetically susceptible individuals (D.K. Podolsky, N Engl J Med 347, 417 (2002)). Genetic factors play an important role in IBD pathogenesis, as evidenced by the increased rates of IBD in Ashkenazi Jews, familial aggregation of IBD, and increased concordance for IBD in monozygotic compared to dizygotic twin pairs (S. Vermeire, P. Rutgeerts, Genes Immun 6, 637 (2005)). Moreover, genetic analyses have linked IBD to specific genetic variants, especially CARD 15 variants on chromosome 16q12 and the IBD5 haplotype (spanning the organic cation transporters, SLC22A4 and SLC22A5, and other genes) on chromosome 5q31 (S. Vermeire, P. Rutgeerts, Genes Immun 6, 637 (2005); J.P. Hugot et al., Nature 411, 599 (2001); Y. Ogura et al., Nature 411, 603 (2001); J.D. Rioux et al., Nat Genet 29, 223 (2001); V.D. Peltekova et al., Nat Genet 36, 471 (2004)). CD and UC are thought to be related disorders that share some genetic susceptibility loci but differ at others.

Onnie Clive M et al. (2008) Eur J Gastrenterology and Hepatolgy Vol 20 No.1 (1) pages 37-45 discloses CARD 15 mutations are associated with the presence of granulomas in CD and Brinar M et al.(2009) J Crohn's and Colitis Vol 3 No. 1(1) page S96 discloses mutations in ATG4 have a role in granuloma formation in CD.

### SUMMARY OF THE INVENTION

Various embodiments include a method of diagnosing susceptibility to granuloma in an individual with Crohn's disease, comprising obtaining a sample from the individual, assaying the sample to determine the presence or absence of at least one risk genetic variant, assaying the sample to determine the presence or absence of at least one risk serological marker, and diagnosing susceptibility to granuloma in the individual if the at least one risk genetic variant is present, or if the at least one risk serological marker is present, or if the at least one risk genetic variant is present and the at least one risk serological marker is present.

According to the present invention there is provided a method of diagnosing susceptibility to granuloma in an individual with Crohn's disease or diagnosing granuloma in an individual with Crohn's disease , comprising:
(a) assaying a sample from the individual to determine the presence or absence of at least one risk genetic variant,
   wherein the at least one risk genetic variant comprises SEQ. ID. NO.: 1 (rs13148469), SEQ. ID. NO.: 2 (rs2050719), SEQ. ID. NO.: 3 (rs7760387), SEQ. ID. NO.: 4 (rs9399527), SEQ. ID. NO. : 5 (rs9784771), SEQ. ID. NO.: 6 (rs282792), SEQ. ID. NO.: 7 (rs10440086), SEQ. ID. NO.: 8 (rs1352851), SEQ. ID. NO. : 11 (rs443394), SEQ. ID. NO.: 12 (rs8091293), SEQ. ID. NO.: 13 (rs10514090), or a combination thereof; and
(b) diagnosing susceptibility to granuloma in the individual if the at least one risk genetic variant is present relative to a healthy individual.

Such risk genetic variant may be at the genetic locus of TGFb3, FTO, NPAS2, MUC1, IL10, LRAP, LRRK2, TNFSF15, or cytochrome P-450 cluster, or a combination thereof.

The sample may also be assayed to determine the presence of absence of at least one risk serological marker (e.g. selected from the group consisting of anti-Cbir1, ANCA, ASCA, anti-OmpC, and anti-I2) ; and wherein diagnosis of susceptibility to granuloma in the individual is if the at least one risk genetic variant is present and the at least one risk serological marker is present, relative to a healthy individual.

It is preferred that the at least one risk serological marker is ASCA and more preferred the ASCA is present in high titre.

The Crohn's disease may be associated with a small bowel disease phenotype, an aggressive complicating phenotype, an internal penetrating disease phenotype, a stricturing disease phenotype, or a fibrostenosing disease phenotype, or a combination thereof.

Other features and advantages of the invention will become apparent from the following detailed description, taken in conjunction with the accompanying drawings, which illustrate, by way of example, various embodiments of the invention.

### BRIEF DESCRIPTION OF THE FIGURES

Figure 1 depicts top hits of various listed SNPs and corresponding alleles as associated with granuloma from performed GWAS.
Figure 2 depicts top hits of various listed SNPs and corresponding alleles as associated with granuloma from performed GWAS.

### DESCRIPTION OF THE INVENTION

Unless defined otherwise, technical and scientific terms used herein have the same meaning as commonly understood by one of ordinary skill in the art to which this invention belongs. Singleton et al., Dictionary of Microbiology and Molecular Biology 3rd ed., J. Wiley & Sons (New York, NY 2001); March, Advanced Organic Chemistry Reactions, Mechanisms and Structure 5th ed., J. Wiley & Sons (New York, NY 2001); and Sambrook and Russel, Molecular Cloning: A Laboratory Manual 3rd ed., Cold Spring Harbor Laboratory Press (Cold Spring Harbor, NY 2001), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described.

"IBD" as used herein is an abbreviation of inflammatory bowel disease.

"CD" as used herein is an abbreviation of Crohn's Disease.

"SNP" as used herein is an abbreviation of single nucleotide polymorphism.

As used herein, the term "biological sample" means any biological material from which nucleic acid molecules can be prepared. As non-limiting examples, the term material encompasses whole blood, plasma, saliva, cheek swab, or other bodily fluid or tissue that contains nucleic acid.

As disclosed herein, the inventors identified clinical, serologic and genetic factors associated with granuloma formation in Crohn's disease (CD). 371 patients with CD who underwent disease-related surgical resection by a single surgeon were included in the study. Surgical samples were examined specifically for the presence or not of granulomas. Patients' demographic and clinical characteristics were collected by chart review, and samples drawn for IBD related serology (ASCA, anti-I2, anti-OmpC, CBirl and ANCA) and genetic analyses. Genome-wide analyses were performed using Illumina technology. Standard statistical tests for association were used and genetic association was assessed both at the genome-wide level and against known IBD and Leprosy susceptibility loci.

As further disclosed herein, 34.7% of CD surgical samples were found to contain granulomas. Granulomas were not associated with CD disease behavior. High ASCA titer was associated with the presence of granulomas (p=0.02). Patients with granulomas were younger at time of surgery (29.9 vs. 37.6 years, p=5x10-7) and far less likely to have ever smoked (12 vs. 32%, p=7x10-5). 14 Single Nucleotide Polymorphisms (SNPs) were associated with granulomas at a level of nominal association at a genome-wide level (p< 0.00005). These include a SNP adjacent to TGFb3, which has been implicated in the pathogenesis of stricturing Crohn's disease, and FTO, which is regulated by oral intake and is associated with raised body mass index. The strongest association was with NPAS2 (p =1x10-6), a core circadian gene that has been shown to modulate transcription of CX3CL1, a chemokine involved in CD pathogenesis. Amongst known IBD-associated loci, 7 were associated with granuloma formation (p<0.05), including: MUC1 (KL-6), also associated with granuloma-forming hypersensitivity pneumonitis; IL10, with known immunoregulatory function in the gut; and LRAP, associated with antigen presentation and LRRK2 a leucine-rich rpeat kinase gene. One TNFSF15 SNP showed a trend towards association with the presence of granulomas (P = 0.066), of particular interest given a recent report that TNFSF15 is associated with Leprosy, another granulomatous condition. Of the known Leprosy loci (in addition to LRRK2 and TNFSF15), the inventors identified association with granulomatous CD and SNPs across the cytochrome P-450 cluster. Thus, the inventors have demonstrated putative genetic and demographic associations with the presence of granulomas in CD including a number of genes associated with Leprosy suggesting unique pathways in the pathogenesis of this subset of CD.

Disclosed herein are methods of diagnosing susceptibility to a subgroup of Crohn's disease in an individual, by obtaining a sample from the individual, and assaying the sample to determine the presence or absence of one or more genetic risk variants and/or risk serological markers, wherein the presence of one or more genetic risk variants and/or risk serological markers is indicative of susceptibility to the subgroup of Crohn's disease. In another embodiment, the subgroup of Crohn's disease is characterized by granuloma manifestations. In another embodiment, the one or more genetic risk variants are at the genetic loci of TGFb3, FTO, NPAS2, MUC1, IL10, LRAP, LRRK2, TNFSF15, and/or cytochrome p450 cluster. In another embodiment, the one or more risk serological markers include a high expression level of ASCA relative to a healthy subject.

A variety of methods can be used to determine the presence or absence of a variant allele or haplotype. As an example, enzymatic amplification of nucleic acid from an individual may be used to obtain nucleic acid for subsequent analysis. The presence or absence of a variant allele or haplotype may also be determined directly from the individual's nucleic acid without enzymatic amplification.

Analysis of the nucleic acid from an individual, whether amplified or not, may be performed using any of various techniques. Useful techniques include, without limitation, polymerase chain reaction based analysis, sequence analysis and electrophoretic analysis. As used herein, the term "nucleic acid" means a polynucleotide such as a single or double-stranded DNA or RNA molecule including, for example, genomic DNA, cDNA and mRNA. The term nucleic acid encompasses nucleic acid molecules of both natural and synthetic origin as well as molecules of linear, circular or branched configuration representing either the sense or antisense strand, or both, of a native nucleic acid molecule.

The presence or absence of a variant allele or haplotype may involve amplification of an individual's nucleic acid by the polymerase chain reaction. Use of the polymerase chain reaction for the amplification of nucleic acids is well known in the art (see, for example, Mullis et al. (Eds.), The Polymerase Chain Reaction, Birkhauser, Boston, (1994)).

A TaqmanB allelic discrimination assay available from Applied Biosystems may be useful for determining the presence or absence of a variant allele. In a TaqmanB allelic discrimination assay, a specific, fluorescent, dye-labeled probe for each allele is constructed. The probes contain different fluorescent reporter dyes such as FAM and VICTM to differentiate the amplification of each allele. In addition, each probe has a quencher dye at one end which quenches fluorescence by fluorescence resonant energy transfer (FRET). During PCR, each probe anneals specifically to complementary sequences in the nucleic acid from the individual. The 5' nuclease activity of Taq polymerase is used to cleave only probe that hybridize to the allele. Cleavage separates the reporter dye from the quencher dye, resulting in increased fluorescence by the reporter dye. Thus, the fluorescence signal generated by PCR amplification indicates which alleles are present in the sample. Mismatches between a probe and allele reduce the efficiency of both probe hybridization and cleavage by Taq polymerase, resulting in little to no fluorescent signal. Improved specificity in allelic discrimination assays can be achieved by conjugating a DNA minor grove binder (MGB) group to a DNA probe as described, for example, in Kutyavin et al., "3'-minor groove binder-DNA probes increase sequence specificity at PCR extension temperature, "Nucleic Acids Research 28:655-661 (2000)). Minor grove binders include, but are not limited to, compounds such as dihydrocyclopyrroloindole tripeptide (DPI,).

Sequence analysis also may also be useful for determining the presence or absence of a variant allele or haplotype.

Restriction fragment length polymorphism (RFLP) analysis may also be useful for determining the presence or absence of a particular allele (Jarcho et al. in Dracopoli et al., Current Protocols in Human Genetics pages 2.7.1-2.7.5, John Wiley & Sons, New York; Innis et al.,(Ed.), PCR Protocols, San Diego: Academic Press, Inc. (1990)). As used herein, restriction fragment length polymorphism analysis is any method for distinguishing genetic polymorphisms using a restriction enzyme, which is an endonuclease that catalyzes the degradation of nucleic acid and recognizes a specific base sequence, generally a palindrome or inverted repeat. One skilled in the art understands that the use of RFLP analysis depends upon an enzyme that can differentiate two alleles at a polymorphic site.

Allele-specific oligonucleotide hybridization may also be used to detect a disease-predisposing allele. Allele-specific oligonucleotide hybridization is based on the use of a labeled oligonucleotide probe having a sequence perfectly complementary, for example, to the sequence encompassing a disease-predisposing allele. Under appropriate conditions, the allele-specific probe hybridizes to a nucleic acid containing the disease-predisposing allele but does not hybridize to the one or more other alleles, which have one or more nucleotide mismatches as compared to the probe. If desired, a second allele-specific oligonucleotide probe that matches an alternate allele also can be used. Similarly, the technique of allele-specific oligonucleotide amplification can be used to selectively amplify, for example, a disease-predisposing allele by using an allele-specific oligonucleotide primer that is perfectly complementary to the nucleotide sequence of the disease-predisposing allele but which has one or more mismatches as compared to other alleles (Mullis et al., supra, (1994)). One skilled in the art understands that the one or more nucleotide mismatches that distinguish between the disease-predisposing allele and one or more other alleles are preferably located in the center of an allele-specific oligonucleotide primer to be used in allele-specific oligonucleotide hybridization. In contrast, an allele-specific oligonucleotide primer to be used in PCR amplification preferably contains the one or more nucleotide mismatches that distinguish between the disease-associated and other alleles at the 3' end of the primer.

A heteroduplex mobility assay (HMA) is another well known assay that may be used to detect a SNP or a haplotype. HMA is useful for detecting the presence of a polymorphic sequence since a DNA duplex carrying a mismatch has reduced mobility in a polyacrylamide gel compared to the mobility of a perfectly base-paired duplex (Delwart et al., Science 262:1257-1261 (1993); White et al., Genomics 12:301-306 (1992)).

The technique of single strand conformational, polymorphism (SSCP) also may be used to detect the presence or absence of a SNP and/or a haplotype (see Hayashi, K., Methods Applic. 1:34-38 (1991)). This technique can be used to detect mutations based on differences in the secondary structure of single-strand DNA that produce an altered electrophoretic mobility upon non-denaturing gel electrophoresis. Polymorphic fragments are detected by comparison of the electrophoretic pattern of the test fragment to corresponding standard fragments containing known alleles.

Denaturing gradient gel electrophoresis (DGGE) also may be used to detect a SNP and/or a haplotype. In DGGE, double-stranded DNA is electrophoresed in a gel containing an increasing concentration of denaturant; double-stranded fragments made up of mismatched alleles have segments that melt more rapidly, causing such fragments to migrate differently as compared to perfectly complementary sequences (Sheffield et al., "Identifying DNA Polymorphisms by Denaturing Gradient Gel Electrophoresis" in Innis et al., supra, 1990).

Other molecular methods useful for determining the presence or absence of a SNP and/or a haplotype are known in the art and useful in the methods of the invention. Other well-known approaches for determining the presence or absence of a SNP and/or a haplotype include automated sequencing and RNAase mismatch techniques (Winter et al., Proc. Natl. Acad. Sci. 82:7575-7579 (1985)). Furthermore, one skilled in the art understands that, where the presence or absence of multiple alleles or haplotype(s) is to be determined, individual alleles can be detected by any combination of molecular methods. See, in general, Birren et al. (Eds.) Genome Analysis: A Laboratory Manual Volume 1 (Analyzing DNA) New York, Cold Spring Harbor Laboratory Press (1997). In addition, one skilled in the art understands that multiple alleles can be detected in individual reactions or in a single reaction (a "multiplex" assay). In view of the above, one skilled in the art realizes that the methods of the present invention for diagnosing or predicting susceptibility to or protection against CD in an individual may be practiced using one or any combination of the well known assays described above or another art-recognized genetic assay.

One skilled in the art will recognize many methods and materials similar or equivalent to those described herein, which could be used in the practice of the present invention. For purposes of the present invention, the following terms are defined below.

### Example - Granuloma

The inventors identified clinical, serologic and genetic factors associated with granuloma formation in Crohn's disease (CD). 371 patients with CD who underwent disease-related surgical resection by a single surgeon were included in the study. Surgical samples were examined specifically for the presence or not of granulomas. Patients' demographic and clinical characteristics were collected by chart review, and samples drawn for IBD related serology (ASCA, anti-I2, anti-OmpC, CBirl and ANCA) and genetic analyses. Genome-wide analyses were performed using Illumina technology. Standard statistical tests for association were used and genetic association was assessed both at the genome-wide level and against known IBD and Leprosy susceptibility loci.

34.7% of CD surgical samples were found to contain granulomas. Granulomas were not associated with CD disease behavior. High ASCA titre was associated with the presence of granulomas (p=0.02). Patients with granulomas were younger at time of surgery (29.9 vs. 37.6 years, p=5x10-7) and far less likely to have ever smoked (12 vs. 32%, p=7x10-5). 14 Single Nucleotide Polymorphisms (SNPs) were associated with granulomas at a level of nominal association at a genome-wide level (p< 0.00005). These include a SNP adjacent to TGFb3, which has been implicated in the pathogenesis of stricturing Crohn's disease, and FTO, which is regulated by oral intake and is associated with raised body mass index. The strongest association was with NPAS2 (p =1x10-6), a core circadian gene that has been shown to modulate transcription of CX3CL1, a chemokine involved in CD pathogenesis. Amongst known IBD-associated loci, 7 were associated with granuloma formation (p<0.05), including: MUC1 (KL-6), also associated with granuloma-forming hypersensitivity pneumonitis; IL10, with known immunoregulatory function in the gut; and LRAP, associated with antigen presentation and LRRK2 a leucine-rich repeat kinase gene. One TNFSF15 SNP showed a trend towards association with the presence of granulomas (P = 0.066), of particular interest given a recent report that TNFSF15 is associated with Leprosy, another granulomatous condition. Of the known Leprosy loci (in addition to LRRK2 and TNFSF15), the inventors identified association with granulomatous CD and SNPs across the cytochrome P-450 cluster. Thus, the inventors have demonstrated putative genetic and demographic associations with the presence of granulomas in CD including a number of genes associated with Leprosy suggesting unique pathways in the pathogenesis of this subset of CD.

## Claims

1. A method of diagnosing susceptibility to granuloma in an individual with Crohn's disease or diagnosing granuloma in an individual with Crohn's disease , comprising:
(a) assaying a sample from the individual to determine the presence or absence of at least one risk genetic variant,
wherein the at least one risk genetic variant comprises SEQ. ID. NO.: 1 (rs13148469), SEQ. ID. NO.: 2 (rs2050719), SEQ. ID. NO.: 3 (rs7760387), SEQ. ID. NO.: 4 (rs9399527), SEQ. ID. NO. : 5 (rs9784771), SEQ. ID. NO.: 6 (rs282792), SEQ. ID. NO.: 7 (rs10440086), SEQ. ID. NO.: 8 (rs1352851), SEQ. ID. NO. : 11 (ras443394), SEQ. ID. NO.: 12 (rs8091293), SEQ. ID. NO.: 13 (rs10514090), or a combination thereof; and
(b) diagnosing susceptibility to granuloma or diagnosing granuloma in the individual if the at least one risk genetic variant is present relative to a healthy individual.

2. The method of claim 1, wherein the sample is also assayed to determine the presence or absence of at least one risk serological marker; and wherein diagnosis of susceptibility to granuloma in the individual is if the at least one risk genetic variant is present and the at least one risk serological marker is present, relative to a healthy individual.

3. The method of claim 2, wherein the at least one risk serological marker is ASCA.

4. The method of claim 3, wherein the ASCA is present in high titre.

5. The method of claim 1, wherein the Crohn's disease is associated with a small bowel disease phenotype, an aggressive complicating phenotype, an internal penetrating disease phenotype, a stricturing disease phenotype, a fibrostenosing disease phenotype, or a combination thereof.

## Patentansprüche

1. Verfahren zum Diagnostizieren der Anfälligkeit für Granulom bei einem Individuum mit Morbus Crohn oder Diagnostizieren von Granulom bei einem Individuum mit Morbus Crohn, das Folgendes beinhaltet:
(a) Testen einer Probe von dem Individuum, um das Vorhandensein oder Nichtvorhandensein von mindestens einer genetischen Risikovariante zu bestimmen,
wobei die genetische Risikovariante SEQ. ID. NO.: SEQ 1 (rs 13148469), ID. NO.: 2 (rs2050719), SEQ. ID. NO.: 3 (rs7760387), SEQ. ID. NO.: 4 (rs9399527), SEQ. ID. NO. : 5 (rs9784771), SEQ. ID. NO.: 6 (rs282792), SEQ. ID. NO.: 7 (rs10440086), SEQ. ID. NO.: 8 (rs1352851), SEQ. ID. NO. : 11 (rs443394), SEQ. ID. NO.: 12 (rs8091293), SEQ. ID. NO.: 13 (rs10514090) oder eine Kombination davon beinhaltet; und
(b) Diagnostizieren der Anfälligkeit für Granulom oder Diagnostizieren von Granulom bei einem Individuum, falls die mindestens eine genetische Risikovariante bezogen auf ein gesundes Individuum vorhanden ist.

2. Verfahren gemäß Anspruch 1, wobei die Probe auch getestet wird, um das Vorhandensein oder Nichtvorhandensein von mindestens einem serologischen Risikomarker zu bestimmen; und wobei die Diagnose der Anfälligkeit für Granulom bei dem Individuum vorliegt, falls die mindestens eine genetische Risikovariante und der mindestens eine serologische Risikomarker bezogen auf ein gesundes Individuum vorhanden sind.

3. Verfahren gemäß Anspruch 2, wobei der mindestens eine serologische Risikomarker ASCA ist.

4. Verfahren gemäß Anspruch 3, wobei der ASCA mit hohem Titer vorhanden ist.

5. Verfahren gemäß Anspruch 1, wobei der Morbus Crohn mit einem Phänotyp einer Erkrankung des Dünndarms, einem aggressiven erschwerenden Phänotyp, einem Phänotyp einer internen penetrierenden Erkrankung, einem Phänotyp einer Strikturerkrankung, einem Phänotyp einer Fibrostenose hervorrufenden Erkrankung oder einer Kombination davon zusammenhängt.

## Revendications

1. Méthode de diagnostic de la vulnérabilité au granulome chez un individu souffrant de la maladie de Crohn ou de diagnostic du granulome chez un individu souffrant de la maladie de Crohn, comprenant :
(a) l'analyse d'un échantillon provenant de l'individu pour déterminer la présence ou l'absence d'au moins une variante génétique de risque,
dans laquelle la ou les variantes génétiques de risque comprennent SEQ. ID. NO.: 1 (rs13148469), SEQ. ID. NO.: 2 (rs2050719), SEQ. ID. NO.: 3 (rs7760387), SEQ. ID. NO.: 4 (rs9399527), SEQ. ID. NO. : 5 (rs9784771), SEQ. ID. NO.: 6 (rs282792), SEQ. ID. NO.: 7 (rs10440086), SEQ. ID. NO.: 8 (rs1352851), SEQ. ID. NO. : 11 (rs443394), SEQ. ID. NO.: 12 (rs8091293), SEQ. ID. NO.: 13 (rs10514090), ou une combinaison de celles-ci ; et
(b) le diagnostic de la vulnérabilité au granulome ou le diagnostic du granulome chez l'individu si la ou les variantes génétiques de risque sont présentes par rapport à un individu sain.

2. Méthode selon la revendication 1, dans laquelle l'échantillon est aussi analysé pour déterminer la présence ou l'absence d'au moins un marqueur sérologique de risque ; et dans laquelle le diagnostic de vulnérabilité au granulome chez l'individu est si la ou les variantes génétiques de risque sont présentes et le ou les marqueurs sérologiques de risque sont présents, par rapport à un individu sain.

3. Méthode selon la revendication 2, dans laquelle le ou les marqueurs sérologiques de risque sont l'ASCA.

4. Méthode selon la revendication 3, dans laquelle l'ASCA est présent à forte teneur.

5. Méthode selon la revendication 1, dans laquelle la maladie de Crohn est associée avec un phénotype de maladie de l'intestin grêle, un phénotype de complication agressive, un phénotype de maladie pénétrante interne, un phénotype de maladie rétrécissante, un phénotype de maladie fibrosténosante ou une combinaison de ceux-ci.
